# EUROPEAN PATENT SPECIFICATION

(11) **EP 1 641 394 B1**
(45) Date of publication and mention of the grant of the patent: **08.08.2012**
(21) Application number: 04749036.2
(22) Date of filing: 21.06.2004
(51) Int. Cl.: A61B 5/103, A61B 10/00, A61C 19/04, A61F 2/02

(54) **METHOD AND ARRANGEMENT RELATING TO TESTING IMPLANTS**
VERFAHREN UND ANORDNUNG IM ZUSAMMENHANG MIT DER PRÜFUNG VON IMPLANTATEN
PROCEDE ET AGENCEMENT DESTINES A TESTER DES IMPLANTS

(30) Priority: 19.06.2003 SE 0301825
(43) Date of publication of application: 05.04.2006
(73) Proprietor: Osstell AB, 415 02 Göteborg (SE)
(72) Inventor: CAWLEY, Peter, London W5 4HU (GB); PETTERSSON, Anders, S-416 54 Göteborg (SE)
(74) Representative: VALEA AB
(86) International application number: PCT/SE2004/000998
(87) International publication number: WO 2004/110272

(56) References cited:
- WO-A1-01/19248
- WO-A1-01/22880
- WO-A1-92/18053
- WO-A1-03/011133
- US-A- 3 355 933
- US-A- 5 518 008
- US-A1- 2002 115 944
- US-A1- 2002 143 268

## Description

### Technical field

The present Invention relates to a method and apparatus for testing an implant attached to a bone of a human or animal subject.

### Background of the invention

The use of implants involves the insertion of a metal fixture Into a prepared hole in the bone. During the healing process, the surrounding bone develops an intimate contact with the implant surface and after a suitable time a prosthesis may be attached to the fixture. Such implants are frequently used in dentistry and in cosmetic surgery.

There is a need for a means of clinically observing the quality of the union between the bone and the implant surface. Implant failures can be caused by errors in placement, and premature or inappropriate loading. A non-destructive test, which could be used before loading the implant would help to reduce failures of this type, and would also enable periodic tests to be carried out on implants which are in use to ensure that they are still satisfactory. The test could also provide a quantitative comparison between different implant systems.

X-rays are sometimes used to test the condition of an implant, but they can only show the presence of gross bone loss around the implant. It is also very difficult to monitor the progress of integration over time with x-rays, since it is difficult to reproduce the viewing position and angle with sufficient accuracy. A different sort of test, albeit a crude one, is to tap the structure attached to the implant with a surgical instrument. This test can only distinguish between satisfactory implants and the most grossly defective systems.

The international patent application no. WO 92/18053, relates to a method of testing an implant attached to a bone of a human or animal subject. The method comprises the steps of bringing a member into contact with the implant; detecting at least one resonance frequency of the member when it is In contact with the implant; and interpreting the detected resonance frequency in terms of the degree of attachment of the implant with respect to the bone. However, the method implies using an analysing unit being in contact with the implant through a wire.

US 3,355,933 and WO 99/46603 relate to measuring arrangements for measuring surface vibrations of a large object. The arrangements presented in these documents are not suitable for small spaces, such as a mouth of a patient.

WO 03011133 relates to a medical implant system. In order to obtain information on physical states of the implant in the environment thereof, a sensor element is embedded into the implant and is connected to a measuring device which determines a physical property of the sensor element or the environment thereof and a modification thereof.

The glass fibers of the implant are connected to a transmission element, for example a conventional transponder, and the implant itself or at a distance from the implant within the body of the patient or on the surface of the body of the patient It can also link to an optical element, which emit light and can receive such a small parabolic mirror, a lens or the like. The document is silence of excitation using the detector.

In US 2002143268, a transducer assembly is used with an apparatus for detecting the stability of a bone implant. The transducer assembly uses one or more transducers to excite vibrations in said bone implant and to detect a response to the vibrations. A coupling is used to detachably connect the one or more transducers to circuitry for driving the vibrations and detecting the response. A memory device is provided within the transducer assembly. The memory device can store one or more parameters read from outside of the transducer assembly via the coupling for controlling the use of the transducer.

In WO 01/19248, which discloses the features of the preamble of claim 1, for the determination of stability of an implant in bone, a vibration effectuating unit is used, which gives a frequency signal A corresponding to the stability. A receiving unit receives this signal and converts it to digital information signal, which relates to the stability. A processing unit receives the digital signal and processes it together with information relating to distortion factors from the current situation, such as implant dimension, surface, material, anatomical site etc. The processing unit gives as output a presentation information, which disregards said factors and therefore representing only the stability. WO 01122880 relates to a device for measuring the mechanical impedance of its surroundings. The device includes a force generator, a force sensor, an accelerometer unit, an electronic unit capable of calculating the impedance of the basis of outputs received from a device component and suitable power and control signal sources.

US 5518008 relates to a dental analyzer for analyzing dental implants includes a dental probe having a probe tip for contacting a patient's dental implant. An accelerometer is coupled to the probe tip. A hammer fired by an actuator against the accelerometer impacts the probe tip against the dental implant which vibrates the dental implant. The accelerometer measures the acceleration time history of the vibrating dental implant. A processor converts the measured acceleration time history of the dental implant into a frequency spectrum from which a diagnosis can then be made regarding the condition of the dental implant.

US 3355933 relates to apparatus for testing mechanical properties and physical characteristics of objects from their vibrations.

US 2002/115944 relates to a distance measurement system. The distance measurement system includes at least one resonant circuit, at least one magnetic element with predetermined magnetic properties, a transmitter operable to transmit an electromagnetic pulse, a receiver operable to detect oscillations emitted by said resonant circuit in response to said electromagnetic pulse, and an analyzer operable to analyze an amplitude envelope property of said oscillations, to thereby determine a distance between the resonant circuit and the magnetic element.

### Summary of the invention

The main object of the present invention is to provide contactless testing apparatus and method of an implant.

Another object of the present invention to provide a non-destructive test, which is capable of giving a reliable indication of the quality and/or extent of the union between an implant and the bone to which it is attached in a contactless way.

Another object of the present invention is to provide a testing arrangement, which is disposable providing a purity aspect to the invention.

For these reasons, a method and arrangement for testing an implant attached to a a bone, are provided. The method comprises the steps defined in claim 1. Preferably the member comprises a cantilever beam.

According to another embodiment of the invention, the member is made of a ferromagnetic material. Thus, the resonance frequency is detected by means of the member disturbing a magnetic field.

Preferably, the implant includes a threaded bore, and the cantilever beam is screwed to or into the implant.

The method further comprises the step of comparing the detected resonance frequency with one or more values for the resonance frequencies of the same or similar members in contact with other implants. In one step, the detected resonance frequency is compared with one or more values, taken at different times, for the resonance frequencies of the same or similar member in contact with the same implant.

The method further includes the steps of exciting the member with a force, detecting the response of the member to the force and deriving an output, which is the ratio of the voltage of the response signal to that of the excitation signal.

The invention also relates to an arrangement for testing an implant attached to a bone. The arrangement is defined in claim 15.

According to most preferred embodiment, the detectable part comprises a magnetic member. Thus, the detector comprises a coil. The arrangement further comprises an amplifier, a processor, and a data store. The signals detected by the detector are amplified by the amplifier and applied as an input to be analysed. The analysed output, which represents a ratio of a response voltage to the excitation, is fed to the processor, which varies the frequency output of the oscillator of the analyser, and stores the results in the data store.

According to another aspect of the invention, the detectable part consists of a ferromagnetic material.

According to another aspect of the invention, the detector comprises a coil for detecting disturbances in an external magnetic field.

Most preferably the member comprises a cantilever beam. Advantageously, the beam is arranged or adapted to resonate at a frequency within the range of about 1 to 20 kHz, preferably about 1 to 10 kHz, and more preferably of the order of about 8 kHz.

For sanitary reasons the member is disposable.

The invention also relates to a disposable implant testing part provided for testing an implant attached to a bone. The disposable implant testing part comprises a detectable part, which can be detected contactless by means of a detector.

### Short description of the drawings

The invention will now be further described, by way of example only, with reference to the accompanying drawings, in which:
- Figure 1: is a schematic cross-sectional view of one illustrative example of an implant testing member and apparatus not falling under the scope of the invention;
- Figure 2: is a schematic cross-sectional view of another example of an implant testing member and apparatus not falling under the scope of the invention;
- Figure 3: is a graphical representation of the hypothetical change in the received amplitude with respect to the frequency of a testing beam according to the examples attached to a typical implant;
- Figure 4: is a graphical representation of the hypothetical change in the relative frequency with respect to the bone modulus of the testing beam according to the examples; and
- Figure 5: is a schematic cross-sectional view of one embodiment of an implant testing member and apparatus according to the invention.

### Detailed description of the preferred embodiments

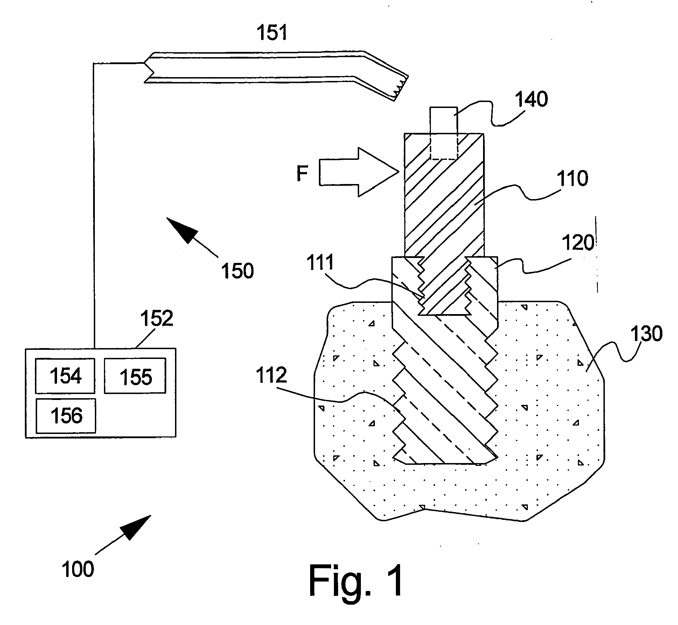

Referring to Figure 1, which shows an illustrative example, the apparatus 100 comprises two parts, a member 110 in the form of a cantilever beam attached by means of a threaded section 111 to an implanted fixture 120. The implant fixture can be a dental implant attached by a threaded section 112 in a section of a bone 130, typically a human jawbone or any other type of an implant for humans or animals. The implant 120 may be any one of a number of known types, formed from a metal, such as titanium, from a ceramic material, or any other appropriate material. It may, for example, be of the type supplied by Nobel Biocare in the U.K. The member 110 is provided with a magnetic member 140. The magnetic member 140 can be provided at one end of the beam 110, e.g. the free end or integrated inside the beam.

The second part of the apparatus comprises the testing apparatus 150, including a probe 151 and a response analyzer unit 152. The probe 150 comprises a coil 153 for detecting oscillations of the magnetic member.

To generate oscillations in the beam, it must be excited. This can be done manually or by means of an electrical exciter, through application of a force F on the beam.

Signals detected by the probe 151 are amplified by an amplifier 154 and applied as an input to the analyser. The output from the analyser, which represents the ratio of the response voltage to the excitation, is fed to a processor such as a microprocessor 155, which is used to vary the frequency output of the oscillator of the analyser, and store the results in a data store 156. The results can be printed out, and/or displayed on a display or the like.

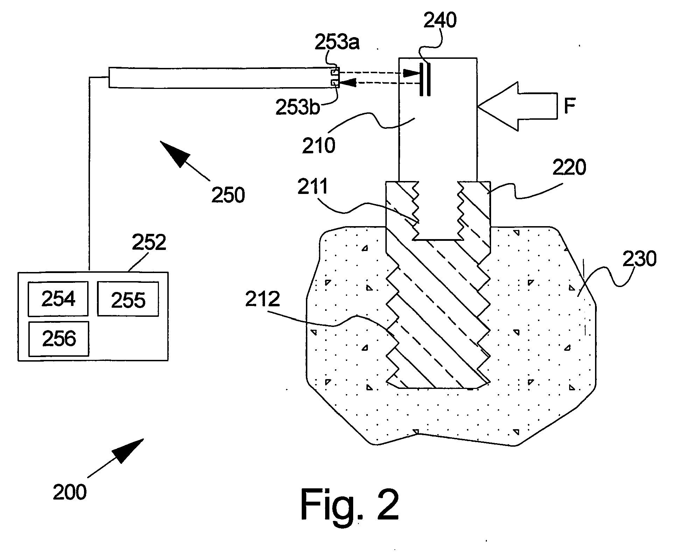

Referring now to Figure 2, illustrating a second example, the first part of the arrangement 200 according to the invention comprises, a member 210 in the form of a cantilever beam as in the earlier embodiment attached by means of a threaded section 211 to the implanted fixture 220. Also, in this case, the implant fixture can be a dental implant attached by a threaded section 212 in a section of a bone 230. The member 210 in this case is provided with markings 240, such as lines, arranged at one end of the beam 210.

The second part of the arrangement comprises the testing apparatus 250, including a probe 251 and a response analyzer unit 252. The probe 250 comprises a light source 253a, preferably but not exclusively a laser, and a light detector 253b for detecting reflections from the beam and thus oscillations of the beam. The light source is preferably Laser diode. The beam is provided with one or several markers, such as darker (or lighter) sections, which effect the reflection of the light.

The beam is excited manually or e.g. by means of an electrical exciter, by applying the force F on the beam.

The light source on the tip of the probe illuminates the beam and the light detector 253b detects the reflected light. The detected light signal is converted to an electrical signal by the detector, and signals detected by the probe 251 are amplified by an amplifier 254 and applied as an input to the analyser. The output from the analyser, which represents the ratio of the response voltage to the excitation, is fed to a processor such as a microprocessor 255, which is used to vary the frequency output of the oscillator of the analyser, and store the results in a data store 256. The results can be printed out, and/or displayed on a display or the like.

In use the beam 110 is secured, i.e. screwed, to the implanted implant 120 with a predetermined torque, for example using a torque controller and counter tool. The variations in resonance frequency with torque have been found to be relatively small over a practical range of torques, for example of the order of 5 to 10 Ncm, so that such torque variations should not present a problem.

Preferably, but not necessarily, the beam is disposable, which means that it can be screwed off and disposed, providing a hygienic testing arrangement.

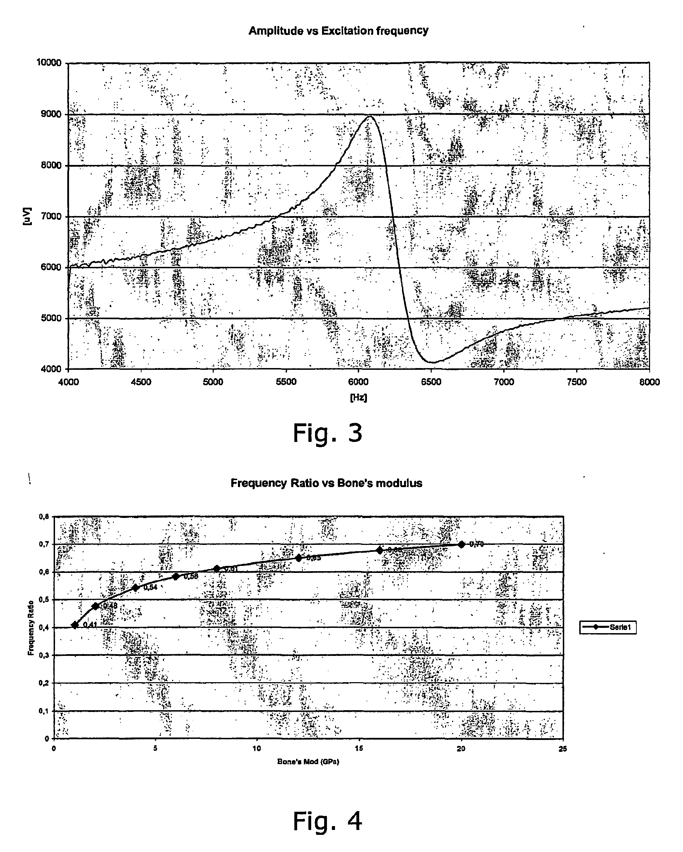

Figure 3 shows the data from a coarse sweep, which is used to obtain the resonance frequency roughly in the apparatus of Figure 1. A finer sweep around this region is then used to identify this frequency, typically the first or fundamental frequency, more accurately. This frequency is noted, and compared, for example, with the data for other implants at similar stages of bonding.

It is expected that for a particular implant, the resonance frequency will vary with the degree of attachment to the bone. Thus by comparing the detected resonance frequency with previously compiled data for similar implants, an indication of the degree of attachment of the implant can be obtained.

The technique, which is based on detection and comparison of resonance frequency shifts, rather than amplitude changes, is effective to determine the quality of the implant/tissue interface as a function of its stiffness, and also in relation to any bone loss as a function of the level or height of the marginal bone surrounding the implant.

The beam is preferably of a metallic material, for example titanium or aluminium, is dimensioned so as to provide a resonant frequency range of the system (placed implant and beam) of the order of 1 to 20 kHz, more specifically 1 to 10 kHz, and preferably in the region of about 8 KHz. For example, in the embodiment of Figure 1, the upright beam can be approximately 1 cm high.

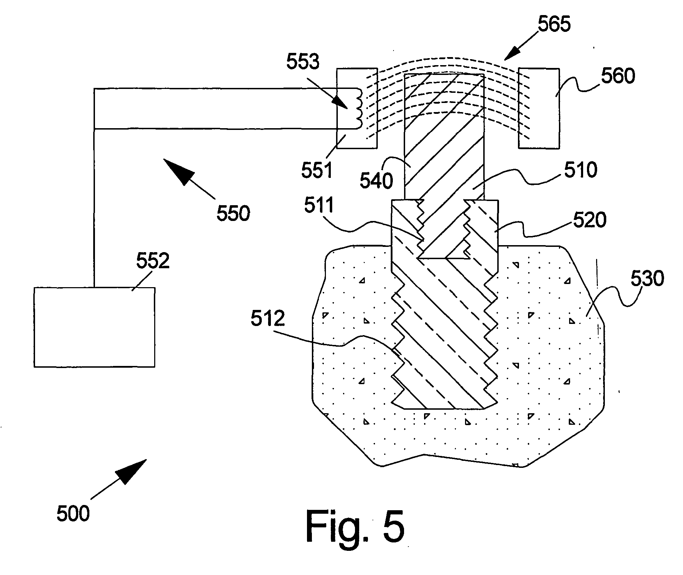

In an embodiment according to the invention, as illustrated in figure 5, the first part of the arrangement 500 according to this aspect of the invention comprises a member 510 in the form of a cantilever beam made of a ferromagnetic material attached by means of the threaded section 511 to the implanted fixture 520. Also, in this case, the implant fixture can be a dental implant attached by a threaded section 512 in a section of a bone 530. Thus, the member 510 in this case is itself the detectable part 540.

The beam 510 is brought into excitation by means of an external magnetic field 565 generated by the field generators 560.

The testing apparatus 550 includes the probe 551 and the response analyzer unit 552. The probe can be part of the magnetic field generator. The probe 550 comprises a coil 553 for detecting interferences in the magnetic field 565. The analysing can be conducted as described in conjunction with the first example.

The field generator can be a permanent magnet for generating a DC field or a coil for generating an AC filed. The probe may also be externally arranged.

It will be understood that various modifications may be made without departing from the scope of the present invention as defined in the appended claims.

The transducers or gauges, and optionally also the beam may be coated, for example with an air-dry acrylic material, to protect the transducers during sterilization of the apparatus. The member may take a form other than a cantilever beam. The beam, instead of being basically straight, could be generally U-shaped, and connected to the implant or abutment by its base. Moreover, alternative detectors, such UV, sound, and the like can also be used.

The invention is limited to implants_{.} The idea might be transferred to all small spaces wherein hold of an object such as screws, rivets, bolt or pin, is to be tested.

## Claims

1. A method of testing an implant (120, 220, 520) attached to a bone (130, 230, 530), the method comprising the steps of:
• attaching a member (110, 210, 510) to said implant by means of a threaded section, • exciting said member,
**characterised by**
• generating said excitation using an external magnetic field,
• contactlessly detecting magnetic field interference of said member to detect at least one resonance frequency of said member (110, 210, 510) during excitation when the member is attached to said attachment (120, 220, 510); and
• analysing the detected the at least one resonance frequency by means of an analyser, output of which represents a ratio of a response voltage to the excitation providing a degree of attachment of the implant (120, 220, 520) to the bone.

2. The method according to claim 1, including the step of detachably attaching said member to said attachment.

3. The method according to claim 1 or 2, wherein said member (110, 120, 520) comprises a cantilever beam.

4. The method according to any of preceding claims, wherein said member (110) comprises a magnetic part (140).

5. The method according to claim 4, wherein said resonance frequency is detected by means of a coil (153).

6. The method according to any of claims 1 to 3, wherein said member (510) is made of a ferromagnetic material.

7. The method according to claim 6, wherein said resonance frequency is detected by means of said member disturbing a magnetic field (565).

8. The method according to claim 3, wherein said attachment includes a threaded bore, and said cantilever beam is screwed to or into the attachment.

9. The method according to any of claims 1 to 8, including the step of comparing the detected resonance frequency with one or more values for the resonance frequencies of the same or similar members in contact with other attachments.

10. A method according to any of claims 1 to 9, including the step of comparing the detected resonance frequency with one or more values, taken at different times, for the resonance frequencies of the same or similar member in contact with the same attachment.

11. A method according to claim 1, including deriving an output which is the ratio of the voltage of the response signal to that of the excitation signal.

12. An arrangement (500) for testing an implants (520) attached to a bone (530), the arrangement comprising:
• a member (510) adapted to be releasably attached to said implant,
• detecting means (550) for detecting at least one resonance frequency of the member when it is attached to the implant,
**characterized by**
an excitation generating part generating a magnetic field (565) or exciting said member, that said member (510) comprises a magnetic detectable part (540) and
that said detecting means (550) comprises a detector (553) for contactless detection of interferences of the magnetic field (565).

13. The arrangements (500) according to claim 12, wherein said detector (55350) comprises a coil (553).

14. The arrangement (500) according to any of claims 12 to 13, further comprising an amplifier (154), a processor (155), and a data store (156).

15. The arrangement (500) according to claim 12, wherein signals detected by the detector (553) are amplified by said amplifier (154) and applied as an input to be analysed; the analysed output, which represents a ratio of a response voltage to the excitation, is fed to said processor, which varies the frequency output of the oscillator of the analyser, and stores the results in said data store (156).

16. The arrangement (500) according to claim 12, wherein said detectable part consists of a ferromagnetic material.

17. The arrangement (500) according to claim 16, wherein said detector (550) comprises a coil (553) for detecting disturbances in an external magnetic field.

18. The arrangement (500) according to any of claims 12 to 17, wherein the member comprises a cantilever beam.

19. The arrangement (500) according to claim 18, wherein the beam is arranged or adapted to resonate at a frequency within the range of about 1 to 20 kHz, preferably about 1 to 10 kHz, and more preferably of the order of 8 kHz.

20. The arrangement (500) according to any of claims 12 to 19, wherein said member (510) is disposable.

## Patentansprüche

1. Verfahren zum Prüfen eines Implantats (120, 220, 520), das an einem Knochen (130, 230, 530) befestigt ist, wobei das Verfahren folgende Schritte umfasst:
- Befestigen eines Bauteils (110, 210, 510) an dem Implantat vermittels eines Gewindeabschnittes,
- Anregen des Bauteils,
**gekennzeichnet durch**
- Erzeugen der Anregung mit einem externen Magnetfeld,
- berührungsfreies Detektieren einer Magnetfeldinterferenz des Bauteils, um wenigstens eine Resonanzfrequenz des Bauteils (110, 210, 510) während der Anregung zu detektieren, wenn das Bauteil an der Befestigung (120, 220, 510) befestigt ist; und
- Analysieren der detektierten wenigstens einen Resonanzfrequenz vermittels eines Analysators, dessen Ausgabe ein Verhältnis einer Antwortspannung zu der Anregung darstellt, das einen Grad der Befestigung des Implantats (120, 220, 520) an dem Knochen bereitstellt.

2. Verfahren gemäß Anspruch 1, das den Schritt aufweist, das Bauteil abnehmbar an der Befestigung zu befestigen.

3. Verfahren gemäß Anspruch 1 oder 2, wobei das Bauteil (110, 120, 520) einen Auslegerbalken umfasst.

4. Verfahren gemäß einem der vorhergehenden Ansprüche, wobei das Bauteil (110) einen magnetischen Teil (140) umfasst.

5. Verfahren gemäß Anspruch 4, wobei die Resonanzfrequenz vermittels einer Spule (153) detektiert wird.

6. Verfahren gemäß einem der Ansprüche 1 bis 3, wobei das Bauteil (510) aus einem ferromagnetischen Material hergestellt ist.

7. Verfahren gemäß Anspruch 6, wobei die Resonanzfrequenz dadurch detektiert wird, dass das Bauteil ein Magnetfeld (565) stört.

8. Verfahren gemäß Anspruch 3, wobei die Befestigung eine Gewindebohrung aufweist und der Auslegerbalken an oder in die Befestigung geschraubt ist.

9. Verfahren gemäß einem der Ansprüche 1 bis 8, das den Schritt aufweist, die detektierte Resonanzfrequenz mit einem oder mehreren Werten für die Resonanzfrequenzen derselben oder ähnlicher Bauteile in Kontakt mit anderen Befestigungen zu vergleichen.

10. Verfahren gemäß einem der Ansprüche 1 bis 9, das den Schritt aufweist, die detektierte Resonanzfrequenz mit einem oder mehreren zu verschiedenen Zeiten genommenen Werten für die Resonanzfrequenzen desselben oder ähnlichen Bauteils in Kontakt mit derselben Befestigung zu vergleichen.

11. Verfahren gemäß Anspruch 1, das ein Ableiten einer Ausgabe aufweist, die das Verhältnis der Spannung des Antwortsignals zu der des Anregungssignals ist.

12. Anordnung (500) zum Prüfen eines Implantats (520), das an einem Knochen (530) befestigt ist, wobei die Anordnung umfasst:
- ein Bauteil (510), das dazu angepasst ist, abnehmbar an dem Implantat befestigt zu sein,
- Detektionsmittel (550) zum Detektieren wenigstens einer Resonanzfrequenz des Bauteils, wenn dasselbe an dem Implantat befestigt ist,
**gekennzeichnet durch**
einen Anregungserzeugungsteil, der ein Magnetfeld (565) zum Anregen des Bauteils erzeugt,
dass das Bauteil (510) einen magnetischen detektierbaren Teil (540) umfasst und
dass das Detektionsmittel (520) einen Detektor (553) zur berührungsfreien Detektion von Interferenzen des Magnetfeldes (565) umfasst.

13. Anordnung (500) gemäß Anspruch 12, wobei der Detektor (553) eine Spule (553) umfasst.

14. Anordnung (500) gemäß einem der Ansprüche 12 bis 13, weiterhin einen Verstärker (154), einen Prozessor (155) und einen Datenspeicher (156) umfassend.

15. Anordnung (500) gemäß Anspruch 12, wobei durch den Detektor (553) detektierte Signale durch den Verstärker (154) verstärkt werden und als ein zu analysierender Eingang angelegt werden; die analysierte Ausgabe, die ein Verhältnis einer Antwortspannung zu der Anregung darstellt, dem Prozessor zugeführt wird, der die Frequenzausgabe des Oszillators des Analysators variiert und die Ergebnisse in dem Datenspeicher (156) speichert.

16. Anordnung (500) gemäß Anspruch 12, wobei der detektierbare Teil aus einem ferromagnetischen Material besteht.

17. Anordnung (500) gemäß Anspruch 16, wobei der Detektor (550) eine Spule (553) zum Detektieren von Störungen in einem externen Magnetfeld umfasst.

18. Anordnung (500) gemäß einem der Ansprüche 12 bis 17, wobei das Bauteil einen Auslegerbalken umfasst.

19. Anordnung (500) gemäß Anspruch 18, wobei der Balken dazu angeordnet oder angepasst ist, bei einer Frequenz innerhalb des Bereiches von circa 1 bis 20 kHz, bevorzugt circa 1 bis 10 kHz und besonders bevorzugt in der Größenordnung von 8 KHz in Resonanz zu stehen.

20. Anordnung (500) gemäß einem der Ansprüche 12 bis 19, wobei das Bauteil (510) ein Einwegteil ist.

## Revendications

1. Procédé de test d'un implant (120, 220, 520) fixé à un os (130, 230, 530), le procédé comprenant les étapes consistant à :
- fixer un composant (110, 210, 510) audit implant au moyen d'une section filetée,
- exciter ledit composant,
**caractérisé par** les étapes consistant à
- générer ladite excitation en utilisant un champ magnétique externe,
- détecter sans contact l'interférence de champ magnétique dudit composant pour détecter au moins une fréquence de résonance dudit composant (110, 210, 510) pendant l'excitation lorsque le composant est fixé à ladite fixation (120, 220, 510) ; et
- analyser l'au moins une fréquence de résonance détectée au moyen d'un analyseur, dont la sortie représente un rapport d'une tension de réponse à l'excitation représentant un degré de fixation à l'implant (120, 220, 520) à l'os.

2. Procédé selon la revendication 1, comprenant l'étape de fixation de façon amovible dudit composant à ladite fixation.

3. Procédé selon la revendication 1 ou 2, dans lequel ledit composant (110, 120, 520) comprend une console.

4. Procédé selon l'une quelconque des revendications précédentes, dans lequel ledit composant (110) comprend une partie magnétique (140).

5. Procédé selon la revendication 4, dans lequel ladite fréquence de résonance est détectée au moyen d'une bobine (153).

6. Procédé selon l'une quelconque des revendications 1 à 3, dans lequel ledit composant (510) est constitué d'un matériau ferromagnétique.

7. Procédé selon la revendication 6, dans lequel ladite fréquence de résonance est détectée au moyen dudit composant perturbant un champ magnétique (565).

8. Procédé selon la revendication 3, dans lequel ladite fixation comprend un alésage fileté, et ladite console est vissée à ou dans la fixation.

9. Procédé selon l'une quelconque des revendications 1 à 8, comprenant l'étape de comparaison de la fréquence de résonance détectée avec une ou plusieurs valeurs pour les fréquences de résonance des composants identiques ou similaires en contact avec d'autres fixations.

10. Procédé selon l'une quelconque des revendications 1 à 9, comprenant l'étape de comparaison de la fréquence de résonance détectée avec une ou plusieurs valeurs, prises à différents temps, pour les fréquences de résonance du composant identique ou similaire en contact avec la même fixation.

11. Procédé selon la revendication 1, comprenant la dérivation d'une sortie qui est le rapport de la tension du signal de réponse à celle du signal d'excitation.

12. Agencement (500) pour tester un implant (520) fixé à un os (530), l'agencement comprenant :
- un composant (510) adapté pour être fixé de façon amovible audit implant,
- des moyens de détection (550) pour détecter au moins une fréquence de résonance du composant lorsqu'il est fixé à l'implant,
**caractérisé par**
une partie de génération d'excitation générant un champ magnétique (565) pour exciter ledit composant, ledit composant (510) comprend une partie magnétique détectable (540) et
lesdits moyens de détection (550) comprennent un détecteur (553) pour la détection sans contact d'interférences du champ magnétique (565).

13. Agencement (500) selon la revendication 12, dans lequel ledit détecteur (553) comprend une bobine (553).

14. Agencement (500) selon l'une quelconque des revendications 12 à 13, comprenant en outre un amplificateur (154), un processeur (155), et un magasin de données (156).

15. Agencement (500) selon la revendication 12, dans lequel les signaux détectés par le détecteur (553) sont amplifiés par ledit amplificateur (154) et appliqués en entrée pour être analysés ; la sortie analysée qui représente un rapport d'une tension de réponse à l'excitation, est transmise audit processeur, qui fait varier la sortie de fréquence de l'oscillateur de l'analyseur, et stocke les résultats dans ledit magasin de données (156).

16. Agencement (500) selon la revendication 12, dans lequel ladite partie détectable est constituée d'un matériau ferromagnétique.

17. Agencement (500) selon la revendication 16, dans lequel ledit détecteur (550) comprend une bobine (553) pour détecter les perturbations dans un champ magnétique externe.

18. Agencement (500) selon l'une quelconque des revendications 12 à 17, dans lequel le composant comprend une console.

19. Agencement (500) selon la revendication 18, dans lequel le faisceau est agencé ou adapté pour résonner à une fréquence dans la plage d'environ 1 à 20 kHz, de préférence d'environ 1 à 10 kHz, et plus préférablement de l'ordre de 8 kHz.

20. Agencement (500) selon l'une quelconque des revendications 12 à 19, dans lequel ledit composant (510) est jetable.
